# EUROPEAN PATENT SPECIFICATION

(11) **EP 4 355 412 B1**
(45) Date of publication and mention of the grant of the patent: **16.09.2026**
(21) Application number: 22738303.1
(22) Date of filing: 10.06.2022
(51) Int. Cl.: A61N 1/04, A61N 1/36

(54) **SYSTEMS FOR MITIGATING PRESSURE INJURIES**
SYSTEME ZUR MINDERUNG VON DRUCKVERLETZUNGEN
SYSTÈMES POUR ATTÉNUER LES BLESSURES DUES À LA PRESSION

(30) Priority: 14.06.2021 US 202163210301 P
(43) Date of publication of application: 24.04.2024
(73) Proprietor: Neurokinetic Medical Inc., Coral Gables, FL 33134 (US)
(72) Inventor: SAMANT, Rahul, Edmonton, AB T5J 4P6 (CA)
(74) Representative: McDonough, Jonathan
(86) International application number: PCT/US2022/072864
(87) International publication number: WO 2022/266591

(56) References cited:
- US-A1- 2007 179 560
- US-A1- 2009 099 627
- US-A1- 2010 004 715
- US-A1- 2011 093 036
- US-A1- 2014 058 476
- US-A1- 2020 353 254
- SANCHEZ BENJAMIN ET AL: "Electrical Impedance Myography and Its Applications in Neuromuscular Disorders", NEUROTHERAPEUTICS, SPRINGER INTERNATIONAL PUBLISHING, CHAM, vol. 14, no. 1, 3 November 2016 (2016-11-03), pages 107 - 118, XP036133154, ISSN: 1933-7213, [retrieved on 20161103], DOI: 10.1007/S13311-016-0491-X

## Description

### FIELD OF DISCLOSURE

Aspects of the present disclosure relate generally to mitigating pressure injuries, and more particularly to, systems, assemblies, and methods for implementing an electrical muscle stimulating system.

### INTRODUCTION

Electrical muscle stimulation (EMS) includes eliciting muscle contractions using electrical impulses. Electrical impulses may be generated by a stimulating device, and delivered to an individual's target muscles through electrodes placed near the muscles. EMS technology has not gained mainstream adoption beyond certain medical, therapeutic, and specialized uses, at least in part because EMS devices can be relatively large, unwieldy, non-portable, and complicated to use. Typically, EMS devices also do not connect with modern, everyday consumer technology such as mobile phones, tablets, and/or wearable devices. EMS devices typically include a controller controlled by software and/or by hardware, such as buttons, knobs, touchscreens, and dials on a hardware interface. The controller may be connected to electrodes via a series of wires. Electrodes may have an adhesive side for contacting and sticking to a patient's body. Electrodes may be single-use or multiple-use. The present disclosure addresses needs that remain generally unmet by EMS technology.
US2007179560A1 relates to an electrical stimulation device for a body part of a person which comprises an orthosis with sensor and electrodes and a controller. The controller receives a sensor signal, compares the sensor signal to a threshold value and generates an electrical output from the electrodes if the sensor signal exceeds the threshold value. The controller comprises a calibration module for determining an initial resting position of the body part.
US2009099627A1 relates to the detection of a movement state of a patient based on brain signals, such as an electroencephalogram (EEG) signal. In some examples, a brain signal within a dorsal-lateral prefrontal cortex of a brain of the patient indicative of prospective movement of the patient may be sensed in order to detect the movement state. The movement state may include the brain state that indicates the patient is intending on initiating movement, initiating movement, attempting to initiate movement or is actually moving. In some examples, upon detecting the movement state, a movement disorder therapy is delivered to the patient. In some examples, the therapy delivery is deactivated upon detecting the patient is no longer in a movement state or that the patient has successfully initiated movement. In addition, in some examples, the movement state detected based on the brain signals may be confirmed based on a signal from a motion sensor.
US2011093036A1 relates to an implantable stimulator for stimulating muscles or nerves, including, an array of electrodes for electrically stimulating muscles or nerves, a controller for controlling the activity of the electrodes, and wherein the controller is adapted to dynamically select the electrodes that are used to participate in stimulating the muscles or nerves.
US2014058476A1 relates to an apparatus and methods for rehabilitating a muscle including one or more electrodes configured to be positioned in or adjacent to tissue, one or more sensors configured to sense muscle contraction and to generate a sensor signal based on the muscle contraction, and a pulse generator operatively coupled to the one or more electrodes, e.g., via a lead, and having a controller configured to receive the sensor signal and to adjust the stimulation frequency based on the sensor signal to cause the muscle to contract where muscle contraction is smooth and continuous.
US2020353254A1 relates to a gait management apparatus that applies stimulation to a user suffering from a neurological disease (such as Parkinson's Disease) gait dysfunction. Motion sensors are arranged to be worn by a patient, and electrical stimulation electrodes are on the legs for stimulation. A controller receives motion sensing signals, and processes these signals to generate stimulation signals for operation of the electrodes to stimulate limb movement upon detection of a gait abnormality. There may be a user actuator for user actuation of electrical stimulation, and the inputs may be a series of taps. The controller may provide signals to prevent occurrence of freezing of gait when it senses that a patient is walking or has an intention to walk. Also, it may apply stimulation at an intensity level which is insufficient for functional muscle stimulation but sufficiently high to trigger activation of efferent nerves.
Document XP036133154 (Neurotherapeutics (2017) 14:107-118 DOI 10.1007/s13311-016-0491-x) relates to Electrical Impedance Myography and Its Applications in Neuromuscular Disorder.

The background description provided herein is for the purpose of generally presenting the context of this disclosure. Unless otherwise indicated herein, the materials described in this section are not prior art to the claims in this application and are not admitted to be prior art, or suggestions of the prior art, by inclusion in this section.

### SUMMARY OF THE DISCLOSURE

According to certain aspects of the disclosure, methods and systems are disclosed for mitigating pressure injuries. Each of the aspects disclosure herein may include one or more of the features described in connection with any of the other disclosed aspects.

According to one example of the present disclosure, an electrical muscle stimulation system includes at least one electrode configured to transmit an electrical current; at least one sensing device configured to detect a physiological characteristic of a user; and at least one controller in communication with the at least one electrode and the at least one sensing device, the at least one controller is configured to activate the at least one electrode in response to the at least one sensing device detecting the physiological characteristic of the user.

**In** some aspects of the present disclosure, the at least one controller is configured to: activate the at least one electrode to deliver the electrical current to a target site in response to determining the physiological characteristic detected by the at least one sensing device exceeds a predetermined threshold; and deactivate the at least one electrode to cease delivery of the electrical current to the target site after a predefined interval. The electrical muscle stimulation system includes a wicking pad coupled to the at least one electrode and the at least one sensing device, the wicking pad includes an interior surface that secures the at least one electrode and the at least one sensing device to the user; wherein the wicking pad is configured transfer moisture from the interior surface of the wicking pad that is in contact with the user to an exterior surface of the wicking pad, thereby drawing moisture away from the user.

In some aspects of the present disclosure, the electrical muscle stimulation system includes a garment having a body that is sized and shaped to be worn by the user, wherein the at least one electrode and the at least one sensing device is coupled to the body; wherein the body is formed of a wicking material such that the garment is configured to remove moisture from the user when the garment is worn by the user. The electrical muscle stimulation system includes a mattress pad having a body that is sized and shaped to receive the user, the mattress pad includes a plurality of electrodes and a plurality of sensing devices on the body; wherein the at least one controller is configured to selectively activate at least a subset of the plurality of electrodes when a corresponding subset of the plurality of sensing devices detects a pressure along the body when the user is received on the mattress pad. The at least one controller is configured to: activate the at least one electrode to deliver the electrical current at a high frequency toward a target site on the user; determine a presence of injured tissue at the target site in response to the at least one sensing device detecting a reflected signal; determine a location of the target site relative to the user based on a measurement of the reflected signal; and deliver the electrical current with a first charge sufficient to stimulate the target site at the location.

According to another example of the present disclosure, an electrical muscle stimulation system includes at least one electrode configured to transmit an electrical current, the at least one electrode having an exterior surface and an interior surface; and a coating disposed along the interior surface of the at least one electrode, the coating includes an adhesive for securing the at least one electrode to a user, and a drug-eluting material for providing a therapeutic effect to the user.

According to another example of the present disclosure, a patient monitoring system includes a plurality of sensing devices configured to detect a physiological characteristic of a user, wherein each of the plurality of sensing devices is attachable to a body of the user; and at least one controller in communication with the plurality of sensing devices, the at least one controller is configured to transmit an alert to a user device in response to at least one of the plurality of sensing devices detecting the physiological characteristic.

In some aspects of the present disclosure, the physiological characteristic includes a pressure measurement relative to a duration that the pressure measurement is detected by the at least one sensing device. The patient monitoring system includes a plurality of electrodes configured to transmit an electrical current, wherein the at least one controller is configured to: activate at least one of the plurality of electrodes to deliver the electrical current toward one or more target sites on the user; determine a presence of moisture adjacent to the one or more target sites in response to at least one of the plurality of sensing devices detecting an impedance from the electrical current delivered by the at least one electrode; and transmit the alert to the user device indicating the presence of moisture adjacent to the one or more target sites. The at least one controller is configured to determine the impedance is less than a predetermined threshold prior to transmitting the alert to the user device.

According to another example of the present disclosure, a method for detecting a deep tissue injury includes transmitting an electrical impulse having a high frequency between a pair of electrodes; detecting a reflected signal that is indicative of the deep tissue injury from transmitting the electrical impulse; determining one or more properties of the reflected signal; and calculating a position of the deep tissue injury relative to at least one of the pair of electrodes.

In some aspects of the present disclosure, the method includes delivering electrical stimulation to the position of the deep tissue injury; terminating delivery of the electrical stimulation for a minimum duration; and retransmitting the electrical impulse with the high frequency between the pair of electrodes after the minimum duration. The electrical stimulation is delivered to the position of the deep tissue injury for a predefined interval based on a depth of the position of the deep tissue injury. The minimum duration for terminating delivery of the electrical stimulation is based on a depth of the position of the deep tissue injury. The one or more properties of the reflected signal includes an amplitude of the reflected signal or a duration between transmitting the electrical impulse and detecting the reflected signal.

According to another example of the present disclosure, an electrical muscle stimulation system includes a first device configured to generate a magnetic field in response to a pressure being applied thereto; and a second device configured to generate an electrical current in response to the first device generating the magnetic field and the second device being positioned proximate to the first device; wherein the pressure is applied to the first device by a body of the user, and the second device is coupled to the body of the user such that the electrical current is delivered to the body of the user when the magnetic field is generated by the first device.

In some aspects of the present disclosure, the first device is coupled to a first accessory that includes an article of furniture, and the second device is coupled to a second accessory that includes an article of clothing positioned on the body of the user; wherein the first accessory is positioned in proximity to the second accessory. The article of furniture includes a plurality of first devices and the article of clothing includes a plurality of second devices; wherein one or more of the plurality of first devices are configured to generate the magnetic field in response to receiving the pressure applied to the article of furniture, and one or more of the plurality of second devices are configured to generate the electrical current in response to being positioned proximate to the magnetic fields generated by the one or more first devices.

In some aspects of the present disclosure, the second device includes an electrode coupled to an exterior of the second device, and the second device is configured to transfer the electrical current to the electrode; wherein the electrode is in contact with the body of the user, and configured to deliver the electrical current generated from the second device to the body of the user. The electrode does not include a lead wire, such that the electrode is configured to stimulate the body of the user wirelessly when the second device is positioned within the magnetic field generated by the first device. The first device includes a first coil that is configured to generate the magnetic field, and the second device includes a second coil that is configured to generate the electrical current.

It is to be understood that both the foregoing general description and the following detailed description are exemplary and explanatory only and are not restrictive of the disclosed embodiments, as claimed.

As used herein, the terms "includes," "including," "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." For such terms, and for the terms "for example" and "such as," and grammatical equivalences thereof, the phrase "and without limitation" is understood to follow unless explicitly stated otherwise. As used herein, the terms "about" and "approximately" are meant to account for variations due to experimental error. All measurements reported herein are understood to be modified by the term "about," whether or not the term is explicitly used, unless explicitly stated otherwise. As used herein, the singular forms "a," "an," and "the" include plural referents unless the context clearly dictates otherwise. Moreover, in the claims, values, limits, and/or other ranges mean the value, limit, and/or range +/- 10%.

### BRIEF DESCRIPTION OF THE FIGURES

The accompanying drawings, which are incorporated in and constitute a part of this specification, illustrate various exemplary embodiments and together with the description, serve to explain the principles of the disclosed embodiments.
FIG. 1 depicts an exemplary environment for an electrical muscle stimulation system, according to one or more embodiments.
FIG. 2 depicts a flow diagram of an exemplary method of using the electrical muscle stimulation system, according to one or more embodiments.
FIG. 3 depicts an exemplary electrode for use by the electrical muscle stimulation system, according to one or more embodiments.
FIG. 4 depicts an exemplary device having a wicking pad for use by the electrical muscle stimulation system, according to one or more embodiments.
FIG. 5A depicts a plurality of devices positioned along a user's arm, according to one or more embodiments.
FIG. 5B depicts a plurality of devices positioned along a user's leg, according to one or more embodiments.
FIG. 6 depicts an exemplary garment having a plurality of electrodes and sensing devices, according to one or more embodiments.
FIG. 7A depicts an exemplary mattress pad with sensors and electrodes, according to one or more embodiments.
FIG. 7B depicts an exemplary mattress pad with sensors and electrodes secured to a mattress, according to one or more embodiments.
FIG. 8 depicts a flow diagram of an exemplary method of using the electrical muscle stimulation system to detect a deep tissue injury, according to one or more embodiments.
FIG. 9 depicts a flow diagram of an exemplary method of using the electrical muscle stimulation system to monitor a patient for incontinence, according to one or more embodiments.
FIG. 10 depicts an exemplary monitoring system for pressure injuries, according to one or more embodiments.
FIG. 11 depicts an exemplary wireless electrode assembly, according to one or more embodiments.
FIG. 12 depicts an exemplary implementation of the wireless electrode assembly of FIG. 11, according to one or more embodiments.

### DETAILED DESCRIPTION

Embodiments of this disclosure relate to electrical muscle stimulation (EMS) systems, devices, assemblies, and methods for eliciting electrical muscle contractions using electrical impulses. The EMS systems described herein may be utilized for various suitable uses, such as, for example, as a rehabilitation and/or preventive tool for immobilized patients, as a strength training tool for healthy individuals to evaluate neuromuscular function, a post-exercise recovery tool, and more.

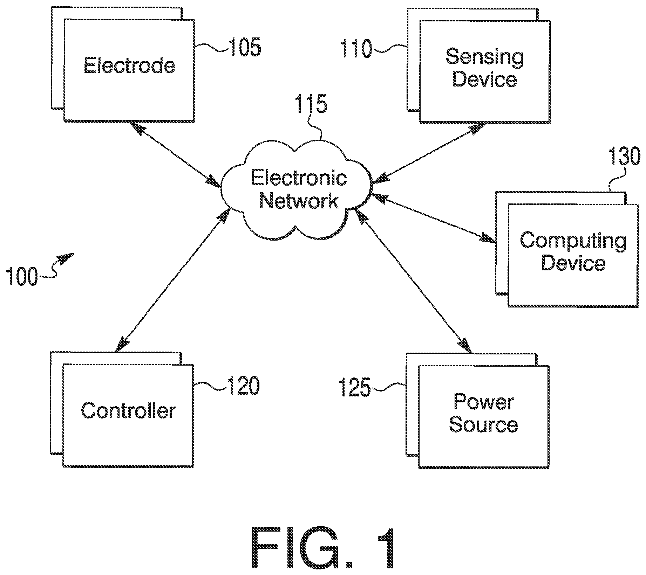

As shown in FIG. 1, an exemplary EMS system 100 disclosed herein may include one or more of an electrode 105, a sensing device 110, a controller 120, a power source 125, and an electronic network 115 facilitating communication between the components of the EMS system 100. Although shown as separate components in FIG. 1, one or more of electrode 105, sensing device 110, controller 120, and/or power source 125 may be combined with one another (e.g., via a connector). Further, a computing device 130 (e.g., a phone, a tablet, a personal computer, or other electronic device) executing a software application may interact with the one or more components of EMS system 100 via electronic network 115 and/or other suitable connection.

EMS system 100 may include a plurality of electrodes 105 in multiple shapes and sizes. For example, electrodes 105 may be integrated in a flexible body that may be adjusted to multiple exercise configurations (i.e., long vs. short muscles). Electrodes 105 may be configured for easy application and reapplication to various surfaces, such as, for example, the skin of a user. Electrodes 105 may be further configured for connection to one or more sensing devices 110 and/or controllers 120 via, for example, a connector and/or electronic network 115. Electrodes 105 may be flexible so as to promote physical comfort during movement.

Still referring to FIG. 1, electrodes 105 may include replaceable electrode pads configured to hold electrodes 105 in place proximal to muscle groups on a user's body that are to be stimulated. Electrode pads may be made of any suitable materials known in the art, including water-based electrode gels, hydrogels, and/or dry (metal) electrodes. In some embodiments, electrode pads may be replaceable, solid electro-gel pads, suitable for application and reapplication to the skin without additional moisturizing or gels. In some embodiments, such electrode pads may include fibers, such as carbon fiber, which may help to preserve the shape and/or flexibility of the electrode pads.

Electrodes 105 may include several layers, such as, for example, the replaceable electrode pads and a conductive material to serve as a lead or leads between controller 120 and electrode pads. In some embodiments, the conductive material may be carbon "black" paper. In further embodiments, the material may include flexible printed circuitry made from a conductive material, such as silver, on a non-conductive substrate. Electrodes 105 may further include a sensing layer, such as, for example, one or more force sensors for detecting pressure, oxygen sensors for detecting oxygen (O₂) levels, infrared (IR) or near infrared (NIR) sensors for detecting electromagnetic measurements at various spectrums, and more.

Electrodes 105 may include other layers, such as protective and/or insulating layers made from non-conductive materials (e.g., materials including polyethylene terephthalate and/or woven polyester), to cover and/or insulate conductive layers, and/or removable protective layers to protect electrode pads when not in use (e.g., removable films, such as plastic films). Electrodes 105 may cover electrical connections between the electrode pads and controller 120, such that no wiring independent of electrodes 105 and controller 120 are visible. Electrodes 105 may therefore include leads from controller 120 to the electrode pads.

Still referring to FIG. 1, sensing devices 110 may include various sensors configured to detect and measure one or more characteristics of a patient. For example, sensing device 110 may include a pressure sensor, a temperature sensor, a moisture meter, an impedance sensor, an ultrasound sensor, a blood-flow sensor, a pulse oximeter (SP02 sensor), and more. As described in detail herein, one or more sensing devices 110 may be operable to detect a physiological measurement of a patient, including but not limited to, tissue oxygenation, surface temperature, surface moisture, electrical impedance, pressure, blood flow, and more. In some embodiments, sensing device 110 may be coupled to electrode 105, such as, for example, in a stacked configuration such that sensing device 110 may be positioned over electrode 105, or vice versa. In other embodiments, sensing device 110 may be integrated into electrode 105 to form a unitary structure with one another. In further embodiments, electrode 105 and sensing device 110 may be separate components positioned adjacent to one another relative to a body of a patient.

Electronic network 115 may include any suitable connection for providing communication between the components of EMS system 100. In some embodiments, electronic network 115 may include one or more wireless connections, including but not limited to, Bluetooth (e.g., Bluetooth Low Energy, Bluetooth 4.0, or Bluetooth SMART), Wi-Fi, or other data connection. In other embodiments, electronic network 115 may include any suitable wired connection, including but not limited to, a USB connector, a micro-USB connector, a USB-c connector, or other cable connection. It should be appreciated that electronic network 115 may include any suitable combination of wireless and wired connections between the components of EMS system 100.

Controller 120 may be optimized for small size, high efficiency in terms of power usage, and/or high performance in stimulation signal intensity and variety, so as to maximize mobility, portability, and performance. In some embodiments, controller 120 may be optimized for a high number of possible exercises between charge cycles. Controller 120 may be configured to receive instructions from, e.g., an application or interface on computing device 130, which may receive inputs from a user. Controller 120 may receive instructions as to the speed, frequency, and/or intensity of desired stimulation at electrodes 105 from the application or interface. Controller 120 may be further configured to interpret such instructions into a series of desired waveforms, and send instructions to generate those waveforms at electrodes 105 by converting the waveforms to electrical pulses that may be output through electrode 105. Alternatively, controller 120 may automatically determine instructions for the speed, frequency, and/or intensity of desired stimulation through electrodes 105 at least partially based on sensor data detected by sensing device 110.

Still referring to FIG. 1, controller 120 may be removably connected to electrodes 105 via a connector, which may include both a physical connection and an electrical connection between an electrical output of controller 120 and conductive material in electrodes 105 that is configured to deliver electrical pulses. A connector may include multiple pieces, with one piece for inclusion on, e.g., controller 120, and another piece for inclusion on electrode 105 or a charger. The electrical connection between an electrical output of controller 120 and electrodes 105 may be, for example, a plurality of conductive pins (e.g., pogo-pins).

EMS systems 100 may be battery-powered, and/or may be powered via a wired connection, such as an A/C cord or a USB connection. For example, power source 125 may include, but is not limited to, rechargeable batteries, such as lithium ion/polymer batteries. Power source 125 may be either replaceable or non-replaceable batteries, and such rechargeable batteries may be charged via a wired connection or a wireless charging protocol.

Still referring to FIG. 1, computing device 130 may be configured to run and/or provide access to one or more applications or interfaces that may control operation of controller 120. Computing device 130 may include a personal user device (e.g., a mobile phone, a tablet, a personal computer, a wearable device, or other device) or a remote device (e.g., a remote server, a cloud computer system, a database, or other computer). An application may be, for example, a mobile device application or set of applications that allow a user to interact with EMS system 100 in a variety of ways. For example, using the application of computing device 130, a user may download, program, edit, revise, select, and/or share stimulation settings, sensor data, short- and long-term EMS regimens, and the like. Computing device 130 may show, for example, a human body muscle diagram that may allow a user to visually identify the muscle group(s) being stimulated by electrodes 105. Alternatively, computing device 130 may allow a user to select (via, e.g., a touch-screen or mouse selection) a muscle group(s) that the user wishes to stimulate. The application may display a variety of stimulation patterns and/or settings pertaining to the selected muscle group(s).

It is contemplated that applications may be useable to program one or more instructions with controller 120, such as to create, revise, and/or program stimulation patterns and/or thresholds for activating stimulation at electrodes 105. Further, the applications may be capable of collecting and recording sensor data (e.g., via sensing device 110) from a user of EMS system 100. In some embodiments, for example, an application may be configured to accept an instruction to record data for a user with EMS system 100 over a given period of time, and conduct one or more operations (e.g., electrical muscle stimulation) based on such data. Additionally, applications according to the present disclosure may be configured to monitor and/or limit usage of EMS system 100 for safety concerns, e.g., by monitoring/limiting the intensity and/or duration of stimulation, monitoring impedance measurements indicative of a lead break, etc.

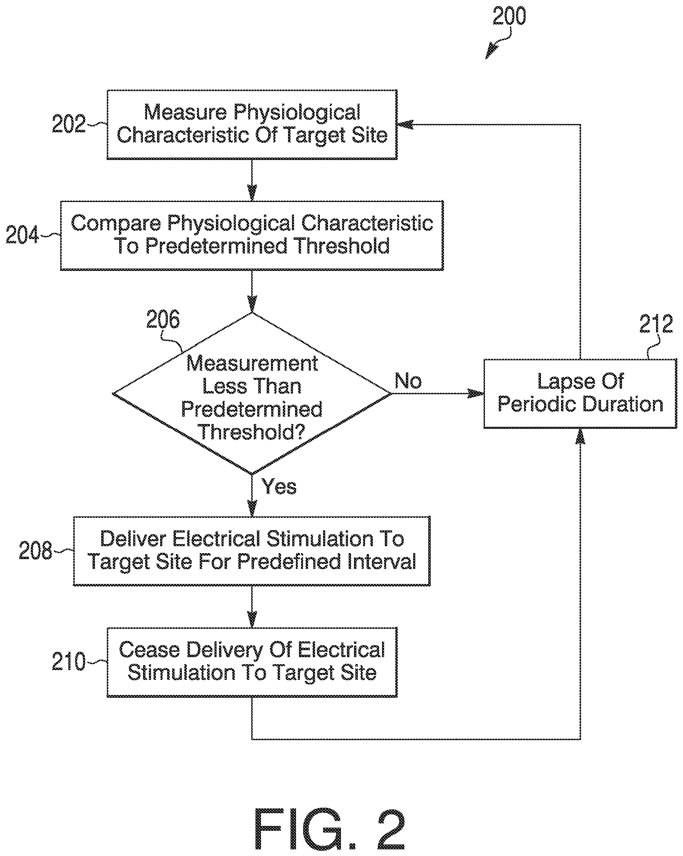

Referring now to FIG. 2, a flow diagram of an exemplary method 200 of using EMS system 100 is depicted. It should be understood that the steps described herein, and the sequence in which they are presented, are merely illustrative such that additional and/or fewer steps may be included without departing from the scope of the present disclosure. It should be appreciated that method 200 may be executed by one or more components of EMS system 100, such as controller 120 and/or computing device 130.

Initially, at least one or more electrodes 105 and sensing devices 110 of EMS system 100 may be positioned along a user's body, and specifically at target site locations that coincide with muscle groups of the user. At step 202, controller 120 may be configured to execute program instructions to measure a physiological characteristic of the user's body at the target site with sensing devices 110. As described in detail above, sensing device 110 may include various suitable sensors for measuring physiological characteristics, such as a pressure over time, a tissue oxygenation, an electrical impedance, a surface temperature, a surface moisture, a blood flow, and more. In some embodiments, sensing device 110 may be configured to measure one physiological characteristic at the target site, while in other embodiments sensing device 110 may measure two or more physiological characteristics.

At step 206, the measurement of the physiological characteristic(s) detected by sensing device 110 may be communicated to controller 120 via electronic network 115, and compared to a predetermined threshold that corresponds to a risk factor for experiencing a pressure injury at the target site. Stated differently, the predetermined threshold may define a measurement of the physiological characteristic that is indicative of a pressure injury occurring at the target site, absent an intervening stimulation of the muscle group located at the target site by EMS system 100.

The predetermined threshold may be predefined, static, or dynamically determined by controller 120. For example, controller 120 may be configured to selectively adjust the predetermined threshold based on user inputs received at EMS system 100, such as, for example, via computing device 130. Alternatively, the predetermined threshold may be determined by controller 120 based on one or more data sources, including but not limited to, a medical history of the patient, recent stimulation regimens (e.g., an intensity, frequency, etc.), and more.

Controller 120 may store a respective predetermined threshold for each of the physiological characteristics measured by sensing device(s) 110. For example, a predetermined threshold for measuring pressure at a target site may range from about 0 millimeters of mercury (mmHg) to about 200 mmHg, such as about 10 mmHg to about 180 mmHg, and particularly about 10 mmHg to about 150 mmHg. A predetermined threshold for measuring tissue oxygenation at a target site may range from about 0 mmHg ptO₂ to about 50 mmHg ptO₂. A predetermined threshold for measuring electrical impedance at a target site may range from about 0 ohms to about 10,000 ohms, such as about 100 ohms to about 5,000 ohms, and particularly about 300 ohms to about 3,000 ohms. A predetermined threshold for measuring skin surface temperature at a target site may range from about 20 degrees Celsius (C) to about 50 C, and about 25 C to about 45 C, and particularly about 30 C to about 40 C. The predetermined threshold for measuring skin surface moisture and/or subdermal moisture may range from about 0 to 1, with a value proximate to 0 being indicative of a relatively low moisture measurement and a value proximate to 1 being indicative of a relatively high moisture measurement. A predetermined threshold for measuring blood-flow of tissue (e.g., using ultrasonic and/or electromagnetic measurements derived from the subject) at a target site may range from about 0 cm/s to about 50 cm/s.

In response to the measurement of the physiological characteristic being greater than (or at least equal to) the predetermined threshold at step 206, controller 120 may be configured to wait for a minimum duration to lapse at step 212 prior to initiating a subsequent measurement of the physiological characteristic (with sensing device 110) at the target site. Controller 120 may be configured to perform a periodic measurement of the physiological characteristic (at step 202) in response to repeatedly determining that the measurement detected by sensing device 110 exceeds (or is equal to) the predetermined threshold at step 206. For illustrative example, the minimum duration for reassessing a measurement of the physiological characteristic at the target site may range from about one (1) second to about two (2) hours, about one (1) minute to about sixty (60) minutes, about five (5) minutes to about thirty (30) minutes, and/or about five (5) minutes to about ten (10) minutes.

In response to the measurement of the physiological characteristic being less than the predetermined threshold at step 206, controller 120 may be configured to deliver electrical stimulation therapy to the target site at step 208 via the corresponding electrode 105 positioned at the target site. Controller 120 may determine an intensity of electrical simulation to transmit based on a deviation of the physiological characteristic measurement from the predetermined threshold. Accordingly, the intensity of electrical current delivered to the target site (via electrode 105) may be at least partially based on a variance measured between the predetermined threshold and the measured physiological characteristic. It should be appreciated that the intensity of electrical current sent to the target site may correlate to an amount of current sufficient to elicit contraction and/or movement of the muscle group(s) at the target site.

In other embodiments, controller 120 may be configured to determine whether the measurement of the physiological characteristic at the target site has declined from a predetermined baseline by a minimum percentage. The predetermined baseline may be predefined, static, or dynamically determined by controller 120. For example, controller 120 may be configured to determine the predetermined baseline based on a user input or an initial (default) measurement of the physiological characteristic of the patient by sensing device 110. Alternatively, the predetermined baseline may be dynamically determined by controller 120 based on one or more data sources (e.g., medical history, stimulation regimens, etc.).

Still referring to FIG. 2, controller 120 may be configured to deliver electrical stimulation at step 208 for a predefined interval, and cease delivery at step 210 upon completion of the predefined interval. In some embodiments, the predefined interval may be stored and/or selectively adjustable. For illustrative example, the predefined interval for delivering electrical stimulation to the target site may range from about one (1) second to about sixty (60) seconds, such as about five (5) seconds to about thirty (30) seconds, and particularly about ten (10) seconds to about fifteen (15) seconds. In other embodiments, the predefined interval may be automatically determined by controller 120 based on the deviation of the physiological characteristic measurement from the predetermined threshold. Accordingly, the duration of electrical stimulation may correlate to the measured variance between the predetermined threshold and the measured physiological characteristic. Upon terminating delivery of electrical stimulation therapy to the target site, controller 120 may be configured to wait the minimum duration at step 212 prior to reevaluating a subsequent measurement of the physiological characteristic of the target site at step 202.

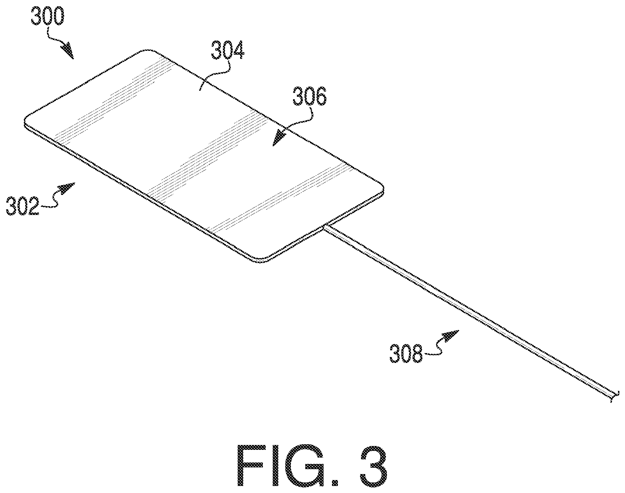

Referring now to FIG. 3, an exemplary electrode 300 is depicted in accordance with an example of the present disclosure. It should be appreciated that electrode 300 may be configured and operable similar to electrode 105 described above except for the differences explicitly noted herein. Accordingly, electrode 300 may be incorporated into EMS system 100 shown and described in detail above.

Electrode 300 may include a body 302 having a first surface 304 and an opposing second surface (not shown). Electrode 300 may include a lead wire 308 extending outwardly from an end of body 302. It should be appreciated that lead wire 308 may include a connector (not shown) at an end opposite of body 302 for facilitating connection between electrode 300 and one or more other components of EMS system 100, such as, for example, controller 120. First surface 304 may define a contact interface of electrode 300 for securing body 302 to a patient, such as, for example, a skin surface of the user. In some embodiments, body 302 may include an adhesive coating 306 positioned along first surface 304 to facilitate fastening electrode 300 to the skin surface of the user.

Still referring to FIG. 3, adhesive coating 306 may be medicated with a drug-eluting material, such that the drug-eluting material may be intermingled with adhesive coating 306. In one embodiment, the drug-eluting material may include a medicinal cream applied to adhesive coating 306. Electrode 300 may be configured to provide a therapeutic effect at the target site as body 302 contacts the skin surface and as the drug-eluting material on first surface 304 is gradually released from body 302. In this instance, the drug-eluting material may permeate through the skin that is in contact with first surface 304 and into the muscle group(s) positioned underneath the skin. By releasing the drug-eluting material from body 302, electrode 300 may be configured to minimize a likelihood of the patient experiencing a pressure injury at the target site. The adhesive coating 306 may include a barrier cream, gel, ointment, and/or paste operable to minimize moisture along the skin and/or breakdown of skin at pressure points along the user's body, and the drug-eluting material may include one or more medicinal ingredients, including but not limited to, dimethicone, petrolatum, zinc oxide, hydrocolloid, alignate, and more.

The drug-eluting material may be further configured to facilitate control of a moisture level of the skin surface to which electrode 300 may be secured. For example, the drug-eluting material may promote a reduction in an existing moisture level of the skin surface upon positioning first surface 304 against the user's body. By reducing the moisture level of the user's skin, electrode 300 may minimize potential interruptions when delivering electrical stimulation to the target site caused by the skin being wet, which may reduce the conductive properties of electrode 105.

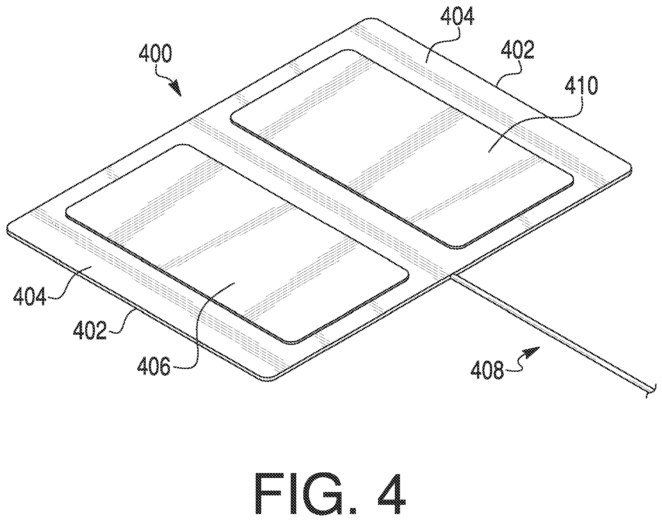

Referring now to FIG. 4, an exemplary device 400 is depicted in accordance with another example of the present disclosure. It should be appreciated that device 400 may be configured and operable similar to one or more of electrode 105 and/or sensing device 110 described above except for the differences explicitly noted herein. Accordingly, device 400 may be incorporated into EMS system 100 shown and described in detail above.

Device 400 may include a body 402 having a wicking pad 404, one or more electrodes 406, and/or one or more sensing devices 410. The one or more electrodes 406 and/or sensing devices 410 may be positioned on wicking pad 404, and each of the electrode(s) 406 and sensing device(s) 410 may be disposed over an exterior surface of wicking pad 404. Wicking pad 404 may be sized and shaped to have a larger profile than each of electrode 406 and sensing device 410. Accordingly, each electrode 406 and sensing device 410 may be encapsulated by wicking pad 404.

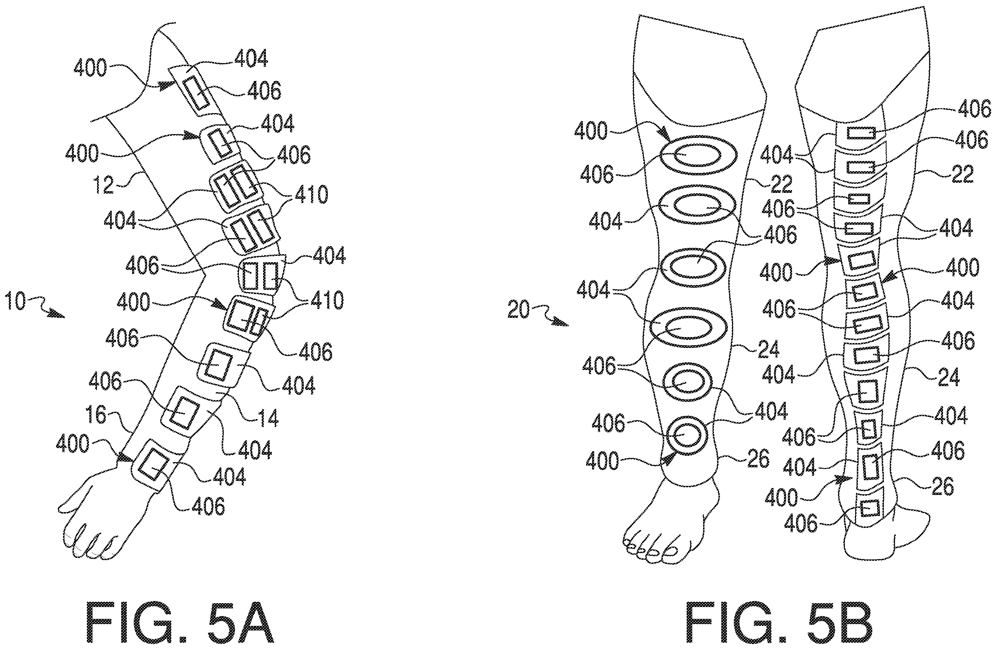

It should be appreciated that wicking pad 404 may have various suitable sizes and/or shapes (see FIGS. 5A-5B). Electrodes 406 may be configured and operable similar to electrodes 105 shown and described above, and sensing devices 410 may be configured and operable similar to sensing devices 110. In other embodiments, device 400 may omit the one or more sensing devices 410 entirely, such that device 400 may only include electrode(s) 406 secured to wicking pad 404.

Body 402 may further include a lead wire 408 extending outwardly from an end of body 402. Lead wire 408 may include a connector (not shown) for facilitating connection between device 400 (e.g., particularly electrode 406 and sensing device 410) and one or more other components of EMS system 100, such as, for example, controller 120. In other embodiments, lead wire 408 may be omitted entirely such that device 400 may be in wireless communication with controller 120 (e.g., via low-energy Bluetooth). Body 402 may be configured to interface with a skin surface of a user along an interior surface of wicking pad 404, to secure device 400 to the user's body. For example, wicking pad 404 may include an adhesive coating disposed along the interior surface for securing device 400 to the user.

Still referring to FIG. 4, wicking pad 404 may be formed of a wicking material operable to draw moisture away from the user's body at the target site. In some embodiments, the wicking material of wicking pad 404 may include a synthetic fiber that is hydrophobic, such that wicking pad 404 may be resistant to the penetration of fluids. For example, the wicking material of wicking pad 404 may include a polyester-based composition, nylon, acrylic, cotton, wool, and more.

Wicking pad 404 may facilitate the transfer of moisture collected on the interior surface of body 402 (e.g., from the body of the user) to an exterior surface of body 402. Wicking pad 404 may further facilitate the drying of body 402 to inhibit moisture generated by the user's body from soaking through device 400. Device 400 may be configured to transfer the moisture (e.g., sweat and/or other bodily fluids) through capillary action. By transferring the moisture from the interior surface of body 402 to the exterior surface, wicking pad 404 may be configured to promote an evaporation of the moisture into the surrounding atmosphere.

As seen in FIGS. 5A-5B, a plurality of devices 400 may be positioned along various areas of a user's body, such as, for example, along one or more target sites that coincide with a location of various muscle groups. For example, devices 400 may be positioned along one or more regions of a user's arm 10, such as an upper arm 12, a lower arm 14, a wrist 16, etc. (see FIG. 5A), one or more regions of a user's leg 20, such as an upper leg 22, a lower leg 24, an ankle 26, etc. (see FIG. 5B), and/or other bodily areas. Wicking pad 404, electrode 406, and/or sensing device 410 may be formed of a flexible material such that body 402 may conform to a curvature and shape of the user's body at the target site. As described in detail above and seen in FIGS. 5A-5B, device 400 may omit sensing device 410 in some embodiments, such that wicking pad 404 may only include one or more electrodes 406 thereon.

The plurality of devices 400 shown in FIGS. 5A-5B include various sizes and/or shapes. As described above, body 402 may have a profile that corresponds to an area of a user's body on which device 400 is to be secured. For example, body 402 may include a squared shape, a rectangular shape, a circular shape, an oval shape, and/or various other shapes. The size and shape of body 402 may be at least partially based on a curvature of the area on the user's body that device 400 is to be secured, to facilitate engagement of device 400 to the user. For example, body 402 may have various sizes and/or shapes depending on a location along the user's arm 10 (e.g., upper arm 12, lower arm 14, wrist 16, etc.) or leg 20 (e.g., upper leg 22, lower leg 24, ankle 26, etc.) where device 400 is to be secured, as seen in FIGS. 5A-5B.

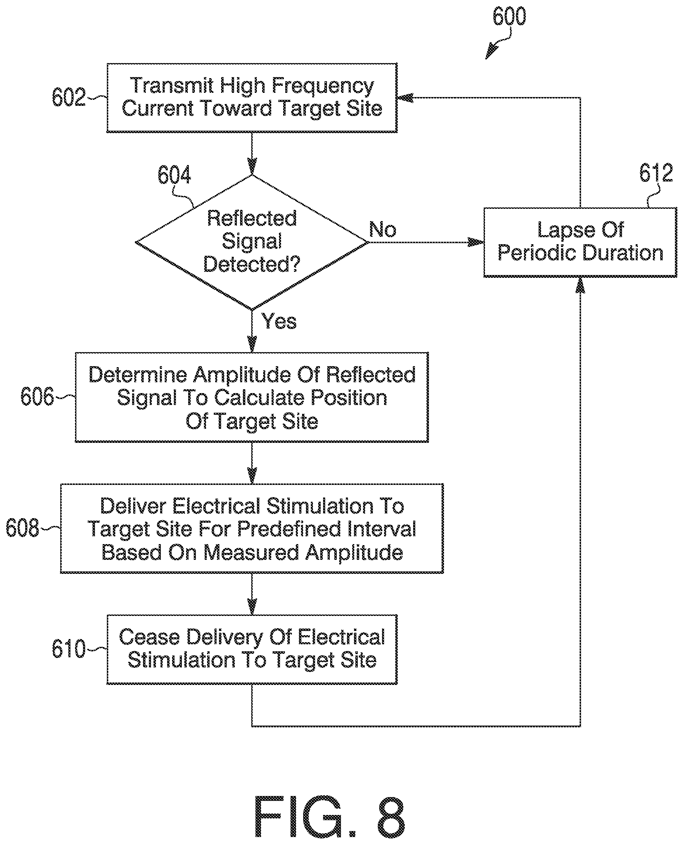

As described in further detail herein, the plurality of devices 400 may form a network that is in communication with one another and a central processing unit, such as, for example, controller 120. Accordingly, controller 120 may be configured to control the network of devices 400 and selectively activate a subset of the plurality of devices 400 based on one or more inputs, such as sensor data received from sensing devices 410 (see FIG. 8).

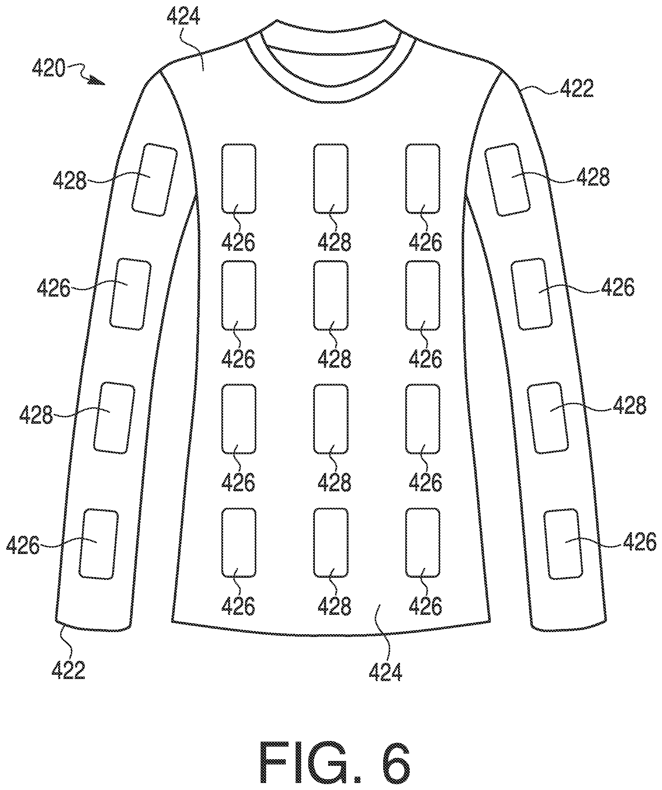

Referring now to FIG. 6, an exemplary garment 420 is depicted in accordance with another example of the present disclosure. Garment 420 may be in the form of various wearable apparatuses, such as, for example, a shirt, a pant, a glove, a sock, a shoe, a hat/cap, and more. In the present example, garment 420 may be sized and shaped in the form of a sweater. Garment 420 may be configured and operable similar to device 400 shown and described above. For example, garment 420 may include a body 422 formed of a wicking material 424 that may draw moisture away from the body of a user wearing garment 420. In some embodiments, wicking material 424 may include a synthetic fiber that is resistance to the penetration of water, such as, for example, a polyester-based composition, nylon, acrylic, cotton, wool, and more.

Wicking material 424 may facilitate the transfer of moisture collected on an interior surface of body 422 (e.g., from the body of the user wearing garment 420) to an exterior surface of body 422. Wicking material 424 may further facilitate the drying of body 422 to inhibit moisture generated by the body of the user from soaking through garment 420. By transferring the moisture to the exterior surface of body 422, garment 420 may promote an evaporation of the moisture into the surrounding atmosphere.

Still referring to FIG. 6, garment 420 may include one or more electrodes 426 and/or one or more sensing devices 428 integrated into body 422, such that each electrode 426 and/or sensing device 428 may be disposed over at least a portion of the wicking material 424. In the present example, garment 420 may include a plurality of electrodes 426 and a plurality of sensing devices 428 positioned at a plurality of locations along body 422. In some embodiments, electrodes 426 and sensing devices 428 may be positioned along body 422 at adjacent locations relative to one another. In other embodiments, electrodes 426 and sensing devices 428 may be disposed over one another in a stacked arrangement, such that each electrode 426 may be positioned atop a corresponding sensing device 428, or vice versa. In this instance, electrode 426 and sensing device 428 may be positioned at an identical location along body 422.

Electrodes 426 and/or sensing devices 428 may be located along portions of body 422 that coincide with areas of a user's body having a greater propensity (high risk areas) for experiencing a pressure injury (e.g., target muscle group(s)). In the present example, with garment 420 being sized and shaped in the form of a sweater, electrodes 426 and/or sensing devices 428 may be positioned along one or more of the sleeves, the chest, the yoke, the collar, the sweep, and/or various other portions of the sweater that may coincide with areas of the body known to endure pressure when garment 420 is worn by the user.

Electrodes 426 may be configured and operable similar to electrodes 105 shown and described above, and sensing devices 428 may be configured and operable similar to sensing devices 110. It should be appreciated that one or more additional components of EMS system 100 may be further integrated into garment 420, such as, for example, controller 120 and/or power source 125. In other embodiments, garment 420 may omit sensing device(s) 428 entirely such that only the plurality of electrodes 416 may be positioned along body 422.

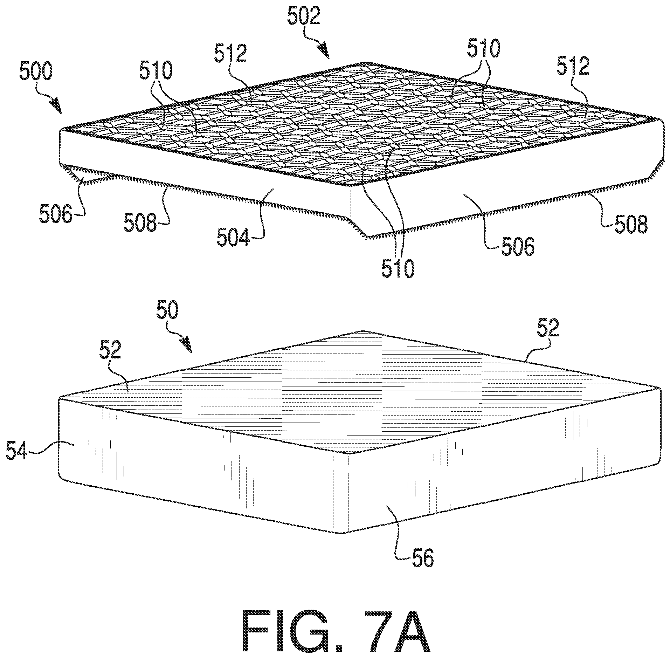

Referring now to FIGS. 7A-7B, an exemplary mattress pad 500 is depicted in accordance with another example of the present disclosure. Mattress pad 500 may include a central top 502, a pair of opposing ends 504, and a pair of sidewalls 506. The ends 504 and sidewalls 506 may be positioned about a perimeter of central top 502, and may collectively define a surface area of central top 502. Mattress pad 500 may include a plurality of electrodes 510 and a plurality of sensing devices 512 positioned across an exterior surface of the central top 502, thereby forming a network of interconnected electrodes 510 and sensing devices 512. Electrodes 510 may be configured and operable similar to electrodes 105 shown and described above, and sensing devices 512 may be configured and operable similar to sensing devices 110.

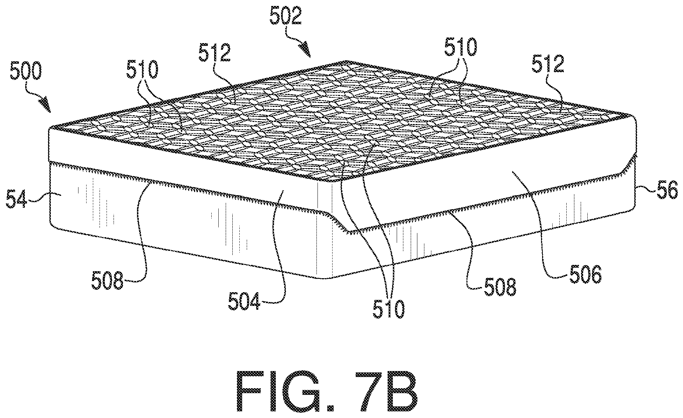

The plurality of electrodes 510 and sensing devices 512 may be positioned along central top 502 in various suitable arrangements. For example, each of the plurality of sensing devices 512 may be stacked atop, integrated into, or positioned adjacent to a corresponding electrode 510 along central top 502. Mattress pad 500 may be sized and shaped to be received over a mattress 50 having a corresponding top 52, pair of opposing ends 54, and sidewalls 56. Mattress pad 500 may include an outer edge 508 extending about the pair of opposing ends 504 and sidewalls 506. The outer edge 508 may be configured to engage the mattress 50, thereby securing mattress pad 500 thereto, as seen in FIG. 7B. In the embodiment, the plurality of electrodes 510 and sensing devices 512 may be positioned along at least central top 502, where the body of a user is received on mattress pad 500. In other embodiments, electrodes 510 and sensing devices 512 may be further positioned on one or more other surfaces of mattress pad 500, such as opposing ends 504 and/or sidewalls 506.

In exemplary use, upon positioning mattress pad 500 onto mattress 50, mattress pad 500 may be operable to continuously and/or periodically measure a pressure received along central top 502 (e.g., via the plurality of sensing devices 512) as a user utilizes mattress 50. The pressure measurements (e.g., sensor data) may be transmitted to controller 120 and a corresponding electrode 510 may be selectively activated by controller 120 in response to determining a pressure measurement is detected at a location of electrode 510. In an example, controller 120 may activate electrode 510 upon detecting a pressure measurement at a target site along central top 502 for a minimum predefined period. For example, the predefined period may range from about one (1) second to about sixty (60) seconds. Therefore, it should be appreciated that controller 120 will activate electrode 510 after a pressure measurement is detected at the target site (via sensing device 512) for at least the predefined period, rather than immediately activating electrode 510 upon detecting pressure.

In some embodiments, controller 120 may be configured to activate the particular electrode 510 corresponding to the sensing device 512 that detected the pressure measurement. Stated differently, controller 120 may selectively activate an individual electrode 510 that is positioned adjacent to and/or stacked atop the sensing device 512 that recorded the pressure measurement. In further embodiments, controller 120 may be configured to activate one or more additional electrodes 510 that may be positioned within a predefined vicinity (relative to a surface area of central top 502) of the sensing device 512 that detected the force from the user's body. In this instance, a greater area of central top 502 may be stimulated by controller 120 to inhibit injury along the user's body at the region that generated the pressure along with the immediately surrounding areas of the user's body.

In other embodiments, controller 120 may be configured to selectively activate one or more of the plurality of electrodes 510 in accordance with the exemplary method 200 of FIG. 2. For example, controller 120 may activate one or more electrodes 510 in response to the corresponding sensing device 512 detecting a pressure measurement that exceeds a predetermined threshold.

Referring now to FIG. 8, a flow diagram of an exemplary method 600 of using EMS system 100 to detect a deep tissue injury is depicted. It should be understood that the steps described herein, and the sequence in which they are presented, are merely illustrative such that additional and/or fewer steps may be included without departing from the scope of the present disclosure.

Initially, at least two or more electrodes 105 and/or sensing devices 110 of EMS system 100 may be positioned along a user's body, and specifically at one or more target site locations coinciding with muscle groups of the user. At step 602, controller 120 may be configured to execute program instructions to transmit an electrical impulse between electrodes 105, and particularly from at least a first electrode 105 (e.g., an anode) and to a second electrode 105 (e.g., a cathode). Electrodes 105 may be positioned such that one or more target sites may be located along the electrical lead path in between the pair of electrodes 105.

The electrical impulse transmitted by first electrode 105 may have a low voltage and high frequency. As described further herein, EMS system 100 may utilize time domain reflectometry to detect deep tissue injuries using time domain reflectometry frequencies ranging from about 100 kilohertz to about 10 gigahertz. In other embodiments, EMS system 100 may transmit radiation at tremendously high frequencies (THF) to the one or more target sites in lieu of an electrical current. In this example, terahertz radiation may be transmitted (e.g., T-rays, T-waves, T-light, T-lux, or THz) at frequencies ranging from about 0.30 terahertz to 30.0 terahertz. Terahertz radiation may include electromagnetic waves that are beyond the visible spectrum.

Through the use of high frequency current, EMS system 100 may be capable of detecting the presence and/or location of deep tissue injuries at a sufficient depth within the user's body using time domain reflectometry. It should be appreciated that tissue experiencing early stages of injury may have varying water content, physical structure, and/or electrical properties relative to healthy tissue. Stated differently, tissue experiencing necrosis may exhibit varying physical, biological, and/or chemical characteristics relative to healthy tissue.

Upon transmitting the high frequency current from first electrode 105 to second electrode 105 at step 602, controller 120 may determine whether a reflected signal from the transmission is detected by sensing device 110, at step 604. Target sites including wounded tissue will present defects and/or obstructions along the electrical path between the pair of electrodes 105, thereby providing an impedance that will cause the signal from the high frequency current to reflect back to a location of first electrode 105 (as detected by an adjacent sensing device 110).

Absent detection of a reflected signal at step 604, controller 120 may be configured to wait for a minimum duration to lapse at step 612 prior to initiating a subsequent transmission of high frequency current toward the target site. Controller 120 may be configured to perform a periodic transmission of current (at step 602) in response to the repeated absence of a reflected signal being detected by sensing device 110 at step 604. For illustrative example, the minimum duration for initiating a subsequent transmission of current toward the target site may range from about ten (10) minutes to about 240 minutes.

In response to detecting a reflected signal at step 604, controller 120 may be configured to determine that a fault is present at the target site, with the fault being indicative of a pressure injury positioned between the pair of electrodes 105. Controller 120 may transmit a notification (e.g., via electronic network 115) altering the user of the presence of the deep tissue injury (e.g., at computing device 130). Alternatively and/or additionally, controller 120 may be configured to determine one or more properties of the reflected signal at step 606 (e.g., an amplitude) to calculate a position (e.g., a depth) of the discontinuity present between electrodes 105 to locate the deep tissue injury. In other embodiments, controller 120 may determine a duration for the reflected signal to be detected at step 604, relative to when the high frequency current was transmitted at step 602, to calculate the position of the injured tissue.

At step 608, controller 120 may be configured to deliver electrical stimulation with a sufficient intensity to reach the target site based on the calculated position (e.g., depth) of the injured tissue. Controller 120 may deliver electrical stimulation for a predefined interval, and cease delivery at step 610 upon completion of the predefined interval. The predefined interval may be stored and/or selectively adjustable between a range of about one (1) second to sixty (60) seconds, such as five (5) seconds to thirty (30) seconds, and particularly ten (10) seconds to fifteen (15) seconds.

In some embodiments, the predefined interval may be automatically determined by controller 120 based on the calculated depth of the injured tissue at step 606. Accordingly, the duration of electrical stimulation may correlate to the measured amplitude of the reflected signal detected at step 604. Upon terminating delivery of electrical stimulation to the target site, controller 120 may be configured to wait the minimum duration at step 612 prior to transmitting a subsequent high frequency current to the target site at step 602.

According to another exemplary method for detecting deep tissue injuries in a patient, metabolomics may be utilized to study specific cellular data of the patient. Initially, one or more specimen samples may be extracted from the patient for analysis. Molecules derived from the specimen samples may be analyzed to determine whether a pressure injury has occurred to tissue within the user's body. For example, specimen samples of the patient's blood, urine, saliva, tissue extracts, etc., may be extracted and analyzed.

Particularly, the cells, bio-fluids, or organisms found in said samples may be inspected to determine the presence of certain metabolic patterns (i.e. chemical processes) that may be indicative of the presence of a pressure injury within the patient. Metabolic profiling from the specimen sample may provide an instantaneous snapshot of the physiology of the patient's biological state. Identification of a metabolic pattern (i.e. chemical footprint) that signifies the existence of a pressure injury may provide early detection of an injury at a stage that may not otherwise be detectable using other devices. For example, the metabolic pattern may be compared to one or more baseline patterns, such that recognition of a discrepancy between the metabolic pattern derived from the specimen sample and the baseline pattern may be indicative of the presence of a pressure injury. The specimen sample may be analyzed using various suitable processes, including but not limited to, a combination of one or more of nuclear magnetic resonance (NMR) spectroscopy, mass spectrometry, highpressure liquid chromatography, ultraviolet (UV) spectroscopy, and more.

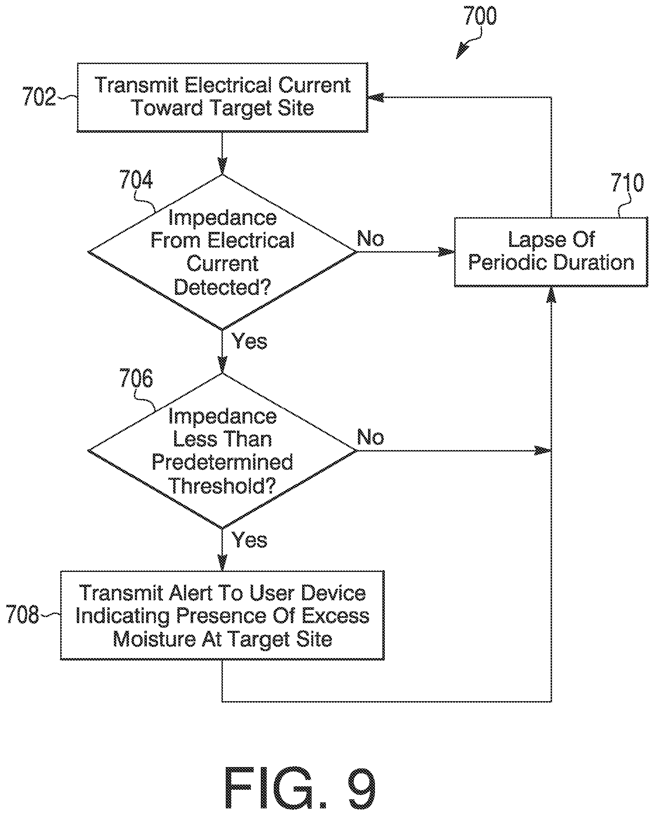

Referring now to FIG. 9, a flow diagram of an exemplary method 700 of using EMS system 100 to monitor a patient for incontinence is depicted. It should be understood that the steps described herein, and the sequence in which they are presented, are merely illustrative such that additional and/or fewer steps may be included without departing from the scope of the present disclosure.

Initially, at least two or more electrodes 105 and/or sensing devices 110 of EMS system 100 may be positioned along a user's body, and specifically at one or more target site locations coinciding with muscle groups of the user. At step 702, controller 120 may be configured to execute program instructions to transmit an electrical current from at least a first electrode 105 and toward a second electrode 105. At step 704, controller 120 may determine whether resistance (i.e. electrical impedance) to the electrical current transmitted at step 702 is detected at sensing device 110.

When no impedance is detected at step 704, controller 120 may be configured to wait for a minimum duration to lapse at step 710 prior to initiating a subsequent transmission of electrical current toward the target site. Alternatively, in other embodiments, controller 120 may wait the minimum duration at step 710 in response to detecting a relatively low electrical impedance at step 704. Controller 120 may be configured to perform a periodic transmission of current (at step 702) in response to sensing device 110 detecting any an absence of and/or low electrical impedance at step 704. **In** the embodiment, a low electrical impedance measurement may range from about 0 ohms to about 1000 ohms. For illustrative example, the minimum duration for initiating a subsequent transmission of current toward the target site may range from about ten (10) minutes to 240 minutes.

**In** response to detecting resistance to the transmitted current at step 704, controller 120 may determine whether the impedance measurement is less than a predetermined threshold at step 706. The predetermined threshold may correspond to a risk factor of the patient experiencing incontinence at the target site. Stated differently, the predetermined threshold may define a measurement of electrical resistance from the current transmitted at step 702 that is indicative of excess moisture being present at the target site adjacent to electrodes 105. The predetermined threshold may be predefined, static, or dynamically determined by controller 120. For example, controller 120 may be configured to selectively adjust the predetermined threshold based on user inputs received at EMS system 100, such as, for example, via computing device 130. Alternatively, the predetermined threshold may be determined by controller 120 based on one or more data sources.

In response to the measured impedance being greater than the predetermined threshold at step 706, controller 120 may be configured to wait the minimum duration to lapse at step 710 prior to initiating a subsequent transmission of electrical current toward the target site. Controller 120 may be configured to perform a periodic transmission of current (at step 702) in response to the impedance detected by sensing device 110 (at step 704) being less than the predetermined threshold.

In response to determining the impedance is less than the predetermined threshold at step 706, controller 120 may be configured to transmit an alert (e.g., via electronic network 115) to a user device at step 708, such as to computing device 130. The alert may indicate the presence of excess moisture at the target site, thereby notifying the user of the need to dry the target site. In other embodiments, step 706 may be omitted entirely such that controller 120 may automatically transmit an alert at step 708 upon detecting any degree of impedance at step 704.

In a further embodiment, controller 120 may be configured to determine a break in the electrical lead path between first electrode 105 (e.g., a cathode) and second electrode 105 (e.g., an anode) in response to detecting the impedance at step 704. It should be understood that damage to the lead assembly between the pair of electrodes 105 may cause various operational problems. Damage in the lead, which may be induced by a break in one or more components of electrodes 105 or changes in the target tissue between the pair of electrodes 105, may also affect the efficacy of the electrical muscle stimulation therapy provided by EMS system 100.

For example, the condition (health) of a lead between electrodes 105 may include a break in the lead and/or an electrical short in a conductor coupled to the lead. Complications resulting from such lead breaks may include, but are not limited to, a degradation of the target tissue adjacent to the lead, corrosion of the lead, contamination or damage to the target tissue adjacent to the lead, pain experienced by the patient, a charge imbalance on electrodes 105, an improper delivery of electrical muscle stimulation to the patient via EMS system 100, and more.

Impedance measurements at step 704 may be used to assess the integrity of the electrical leads of EMS system 100 that deliver the stimulation provided by electrodes 105. A change in the impedance across the leads that deliver the electrical pulses may be indicative of changes in a patient's body (e.g., injured tissue, incontinence, etc.), as described above, and/or changes in the electrical leads themselves. Controller 120 may be configured to determine that an increase or decrease in the controlled current delivered by electrodes 105, beyond the predetermined threshold at step 706, may be indicative of unusually low (or high) lead impedances, which may be indicative of a lead break (or electrical short associated with the lead).

In this instance, controller 120 may be configured to include information identifying the detected change in the measured impedance, which may be indicative of various changes or malfunctions to the lead, in the alert transmitted at step 708. Maintaining integrity of the leads between electrodes 105 that deliver stimulation ensures proper therapy dosages are delivered to the patient. In some embodiments, in response to controller 120 determining a break in the lead (or a constant lead short) between electrodes 105, controller 120 may be configured to prevent further delivery of therapeutic electrical current by electrodes 105 until adjustments to the leads are completed by the user of EMS system 100.

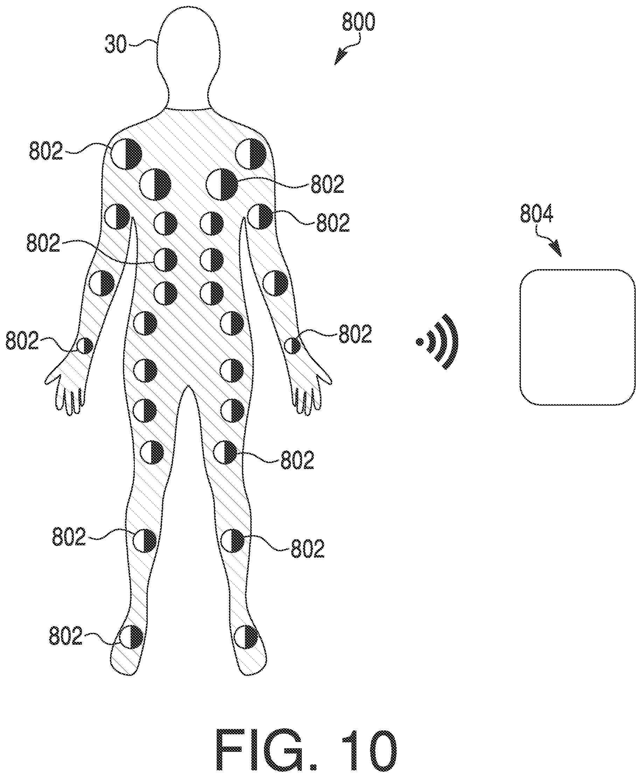

Referring now to FIG. 10, an exemplary monitoring system 800 is depicted in accordance with another example of the present disclosure. System 800 may include a plurality of sensing devices 802 and a controller 804 that is in communication (e.g., wired or wireless connection) with the plurality of sensing devices 802. The plurality of sensing devices 802 may be selectively attached to a body 30 of a user at one or more target sites coinciding with certain muscle groups. Each of the plurality of sensing devices 802 may be configured to detect and measure a pressure applied to the body 30 at the target site, and a duration for which the pressure is applied.

Controller 804 may be configured and operable similar to controller 120 shown and described in detail above. In the present example, controller 804 may receive the sensor data (e.g., pressure and duration measurements) from the plurality of sensing devices 802 in real-time as the body 30 experiences an application of pressure at one or more of the target sites. Controller 804 may be configured to determine areas along the body 30 that may be at increased risk of experiencing a pressure injury based on the sensor data retrieved from the plurality of sensing devices 802.

For example, controller 804 may correlate the pressure measurement to the duration measurement to determine a resulting risk factor score. Controller 804 may compare the risk factor score to a predefined baseline to determine whether the associated target site at which the pressure was detected is at risk of a pressure injury. In other examples, controller 804 may compare the separate measurements of pressure and duration to respective tolerance levels to determine whether at least one of the measurements is indicative of a pressure injury occurring at the target site.

Still referring to FIG. 10, controller 804 may be configured to detect the presence of a pressure injury when determining a target site received a relatively high degree of force, even if the force was applied over a relatively short duration (e.g., seconds). Additionally and/or alternatively, controller 804 may detect the presence of a pressure injury when determining the target site received a relatively low degree of force over a relatively long duration (e.g., minutes). Controller 804 may provide continuous monitoring of the user, and may generate notifications when determining the risk factor score (or individual measurements) are indicative of a pressure injury on the body 30. The notification may include information identifying a location of the target site (i.e. the corresponding muscle group along the body 30) where the potential pressure injury is located. Controller 804 may transmit the notification to the user (e.g., at computing device 130) via electronic network 115.

Controller 804 may further determine a cumulative measurement of the pressure (i.e. application of force) and/or duration that each muscle group on the body 30 has endured. Controller 804 may be configured to generate notifications of the accumulated measurements for transmission to the user (e.g., via electronic network 115). Controller 804 may further prioritize notifications to the user based on the target sites having a greater likelihood of experiencing a pressure injury based on the cumulative measurements calculated at each muscle group.

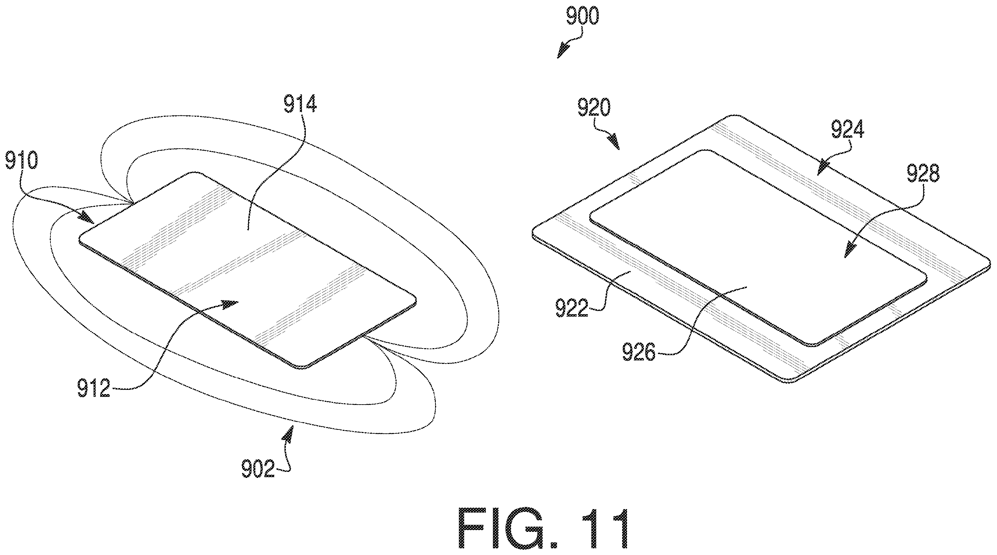

Referring now to FIG. 11, an exemplary wireless electrode assembly 900 is depicted in accordance with another example of the present disclosure. Wireless electrode assembly 900 may include one or more devices, such as, for example, at least one first portion 910 (e.g., a sensing device) and at least one second portion 920 (e.g., an electrode device). As described herein, wireless electrode assembly 900 may include additional first portions 910 and/or second portions 920, with each portion including a device that may be integrated and/or positioned on one or more surfaces, objects, clothing, furniture, and more. It should be appreciated that first portion 910 and second portion 920 of wireless electrode assembly 900 may be configured and operable similar to one or more of electrode 105 and/or sensing device 110 of EMS system 100 described above, except for the differences explicitly noted herein. Accordingly, wireless electrode assembly 900 may be incorporated into EMS system 100.

First portion 910 may include a body 912 having a pair of opposing exterior surfaces 914. In the example, body 912 may be generally rectangular and the pair of opposing exterior surfaces 914 may be generally planar. However, in other embodiments, body 912 and/or the pair of exterior surfaces 914 may have various other suitable sizes, shapes, and/or configurations (e.g., circular, square, polygonal, etc.) than those shown and described herein without departing from a scope of this disclosure. In some embodiments, first portion 910 may include a first magnetic coil (not shown) that is disposed in body 912, such as between the pair of opposing exterior surfaces 914. The first magnetic coil of first portion 910 may be configured to generate a magnetic field 902 about body 912 in response to one or more stimuli and/or events.

For example, first portion 910 (and particularly the first magnetic coil) may be configured to generate magnetic field 902 in response to receiving an electrical current at first portion 910. In some examples, first portion 910 may receive an electrical current, and thereby generate magnetic field 902, in response to experiencing an application of force and/or pressure onto body 912. As described below, one or more first portions 910 may be embedded within, or coupled to, any suitable accessory, such as a mattress, a pillow, a cushion, a chair, a ground surface, a floor mat, an article of clothing, an article of furniture, and various other accessories and/or objects.

An electrical current generated from the force and/or pressure applied to body 912 may travel through and/or be received by first portion 910. In other embodiments, an electrical current may be generated and delivered to first portion 910 from various other suitable sources without departing from a scope of this disclosure. For example, as described below with respect to FIG. 11, a user (e.g., a patient) sitting on a cushion including one or more first portions 910 embedded therein may induce a current through the one or more first portions 910 due to the force and/or pressure applied to the cushion by the user's body seated thereon.

Still referring to FIG. 11, second portion 920 may include a body 922 having a pair of opposing exterior surfaces 924 and an electrode 926 coupled to body 922. In some embodiments, electrode 926 may be positioned along at least one of the pair of opposing exterior surfaces 924. Electrode 926 may include an exterior surface 928 facing relatively outward and away from body 922. It should be appreciated that electrode 926 may be configured and operable similar to electrode 105 described above except for the differences explicitly noted herein. In the example, electrode 926 may not include a lead wire. As described herein, electrode 926 may be configured to stimulate a body of a user wirelessly when second portion 920 is positioned in proximity to first portion 910, and particularly within magnetic field 902 of first portion 910. In some embodiments, electrode 926 may include a coating, a film, and/or other features, such as on exterior surface 928. The coating, as discussed in further detail above, may include an adhesive for securing electrode 926 to a user and/or to an accessory (e.g., an article of clothing). **In** other embodiments, body 922 may include a coating (e.g., an adhesive), such as along one or more of the exterior surfaces 924 for securing second portion 920 to the user.

Second portion 920 may include a second magnetic coil (not shown) that is disposed within body 922, such as between the pair of opposing exterior surfaces 924. The second magnetic coil of second portion 920 may be configured to generate an electrical current in response to one or more stimuli and/or events. For example, second portion 920 (particularly the second magnetic coil) may be configured to generate the electrical current in response to detecting an adjacent magnetic field in proximity to second portion 920, such as magnetic field 902 from first portion 910. With electrode 926 coupled to second portion 920, second portion 920 may be configured to transfer the generated electrical current to electrode 926. As described herein, when wireless electrode assembly 900 is in use, second portion 920 may be positioned on a user such that electrode 926 may be configured to deliver the electrical current to a body of the user. Although second portion 920 is shown and described herein as including a single electrode 926, it should be appreciated that additional electrodes 926 may be included on second portion 920 without departing from a scope of this disclosure.

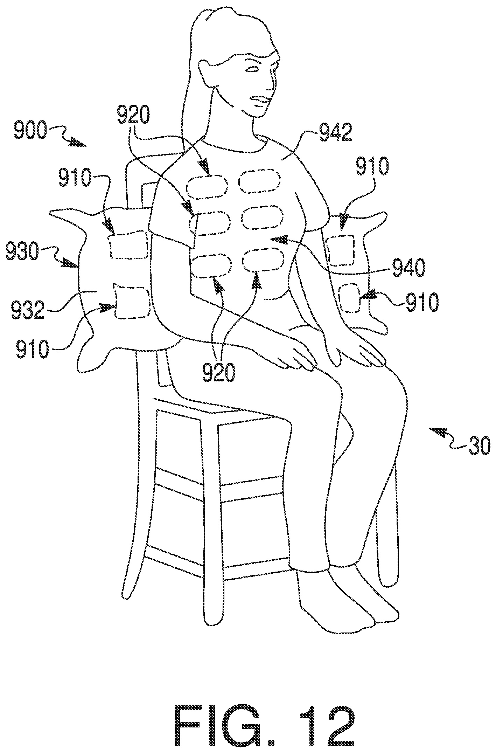

In exemplary use, as seen in FIG. 12, wireless electrode assembly 900 may include a plurality of first portions 910 and a plurality of second portions 920. For example, a plurality of first portions 910 may be embedded in a first accessory 930 and a plurality of second portions 920 may be embedded in a second accessory 940. As described above, each of first accessory 920 and/or second accessory 940 may include various suitable objects, surfaces, clothing, furniture, etc. In the example, first accessory 930 may include an article of furniture 930 (e.g., a pillow or seat cushion), and second accessory 940 may include an article of clothing 940 worn by the user (e.g., a shirt or a sweater). The plurality of first portions 910 may be positioned along an exterior surface 932 of first accessory 930 and/or embedded within first accessory 930. The plurality of second portions 920 may be positioned along an exterior surface 942 of second accessory 940 and/or embedded within second accessory 940.

In another embodiment, at least a portion of the plurality of second portions 920 may include an adhesive, as discussed above, such as along exterior surface 922 and/or exterior surface 928 of electrode 926. In this instance, the plurality of second portions 920 may be configured to adhere to second accessory 940 (e.g., along exterior surface 942, or an interior surface of second accessory 940), and/or directly onto the user's body 30. With the user wearing second accessory 940 and seated against first accessory 930, the user's body 30 may apply a force and/or pressure onto at least a portion of first accessory 930, e.g., by leaning against the article of furniture (e.g., the pillow or seat cushion of first accessory 930) as depicted in FIG. 12. The force and/or pressure applied onto first accessory 930 may be received along at least one or more of the plurality of first portions 910, thereby inducing an electrical current at the respective first portions 910. Stated differently, the electrical current generated and/or induced at each of the first portions 910 (e.g., by the first magnetic coil) may create magnetic field 902 about the respective body 912 of each first portion 910.

Still referring to FIG. 12, each of the plurality of first portions 910 receiving an application of force and/or pressure thereon may be configured to generate a corresponding magnetic field 902 about a respective body 912 of the first portion 910. With second accessory 940 positioned adjacent to first accessory 930, one or more of the plurality of second portions 920 may be located in proximity to one or more of the magnetic fields 902 created by the one or more first portions 910. The one or more second portions 920 positioned in proximity to one or more of the magnetic fields 902 may be configured to generate an electrical current that moves through the respective body 922 of the second portion 920 and into the corresponding electrode 926 coupled thereto. Stated differently, the electrical current generated and/or induced by second portion 920 (e.g., by the second magnetic coil) may be transferred to electrode 926.

In some embodiments, second portion 920 may be positioned in proximity to first portion 910 when second portion 920 is within about one or more millimeters, one or more centimeters, or one or more inches from first portion 910. It should be appreciated that first portion 910 and second portion 920 may be separate devices from one another, such that wireless electrode assembly 900 may be configured to generate the electrical current at second portion 920 (and particularly at electrode 926) wirelessly from first portion 910. Stated differently, the electrical current transferred to electrode 926 from second portion 920 may be generated locally and not received by an ancillary device (e.g., a stimulator, a power source, etc.) and/or from first portion 910.

Still referring to FIG. 12, with the plurality of second portions 920 embedded in second accessory 940, one or more electrodes 926 may be configured to deliver the electrical current to a target site on the user's body 30, thereby stimulating one or more areas (e.g., muscles) of the user's body 30. In the embodiment, the plurality of second portions 920 may be coupled to second accessory 940 along a rear exterior surface 942, such that electrodes 926 may be configured to deliver the electrical current to a rear area (e.g. a backside) of the user's body 30. In some embodiments, the one or more electrodes 926 may be configured to deliver the electrical current to the target site for a predefined interval. In other embodiments, the one or more electrodes 926 may be configured to deliver the electrical current to the target site until the force and/or pressure applied against first accessory 930, and particularly the plurality of first portions 910 embedded therein, ceases. It should be appreciated that the plurality of second portions 920 may be positioned along various suitable locations relative to the user's body 30 (e.g., a lower body, an upper body, a front area, a shoulder, a calf, a foot, a hand, etc.).

In other embodiments, the one or more systems, assemblies, and/or devices described above (e.g., EMS system 100) may be configured and operable to detect a muscle contraction and determine an electrical stimulation intensity at least partially based on the detected muscle contraction. In the embodiment, sensing device 110 may include an accelerometer and/or force sensor operable to detect movement of a muscle indicative of a contraction. Sensing device 110 may further measure a force produced by the muscle movement and/or redistribution of muscle shape (e.g., by an ancillary object). In this instance, EMS system 100 may deliver electrical stimulation at an intensity level that corresponds to (e.g., is proportionate to) the measured force.

It should be appreciated that EMS system 100, and particularly the one or more systems, assemblies, and/or devices described above, may be used in a variety of ways. For example, EMS system 100 according to the present disclosure may be suitable for use in a home, in a doctor's office, a hospital, a medical care facility, and/or an athletic training facility. EMS system 100 may be suitable for use by healthy, able-bodied individuals and/or individuals undergoing treatment to rehabilitate and/or strengthen muscles in their bodies.

EMS systems according to the present disclosure may be used to stimulate one or more of a wide variety of muscle groups. Some examples of muscle groups that the EMS systems may stimulate include: abdominal muscles, upper arm muscles (e.g., biceps and triceps), shoulder muscles, gluteal muscles, lateral muscles, lower back muscles, trapezius muscles, abductors, calf muscles, forearm muscles, hamstrings, quadriceps, and pectoral muscles. EMS systems according to the present disclosure may be used for physical therapy, rehabilitation, athletic training, and/or to prevent medical conditions or illnesses (e.g., pressure ulcers or deep vein thrombosis).

In some embodiments, multiple EMS systems may be used on a body at once, to stimulate and/or monitor different muscles. (See, e.g., FIGS. 1-12.) In some embodiments, multiple EMS devices may be controlled via a single application. For example, in some embodiments, two EMS devices may perform coordinated electrical stimulation and/or pressure monitoring on two different parts of the body (e.g., mirror-image stimulation on two sides of the body). In some such embodiments, multiple EMS devices may be linked together in a network (e.g., a mesh network via a wireless connection) in order to, e.g., stimulate muscles of a patient in a given pattern. Multiple EMS devices may be coordinated in a mesh network in order to create and/or monitor electrical stimulation of a patient's body, e.g., to simulate muscle usage. Such coordination may be controlled by, e.g., a single application linked to the multiple EMS devices. In further embodiments, multiple systems in a linked network may be attached to a single, larger electrode having multiple leads, in order to create and/or monitor muscle stimulation in a given pattern.

In some embodiments, data may be gathered from and/or distributed to multiple EMS systems each having one or more controllers. Data gathered from multiple EMS systems may be used, e.g., as a basis to generate more complex stimulation patterns, to create or modify stimulation patterns based on a larger data set, and/or to serve as a basis for machine learning. For example, data gathered from multiple EMS systems or multiple controllers may, over time, show trends as to the safety and/or effectiveness of stimulation patterns. Such data may be used to generate new, improved stimulation patterns, pressure monitoring patterns, or update existing stimulation and/or monitoring patterns. Such data may also be used to inform training decisions and therapy decisions. Also, data may be distributed to multiple EMS systems, e.g., in order to distribute stimulation patterns, update stimulation patterns, update safety protocols, etc.

In some embodiments, an EMS device may include more than two leads, such as three, four, five, or six leads, which all may be managed by a single application. In some embodiments, an application may be configured to manage EMS systems having varied numbers of electrodes, electrode leads, sensing devices, etc. In some embodiments, EMS systems may be integrated in other types of apparatuses, apparel, physical therapy devices, or medical equipment. It is contemplated that the components of EMS system 100 shown and described herein (e.g., electrodes, sensing devices, controller, etc.) may be integrated into, e.g., such apparel or other therapy devices (such as bandages, slings, compression socks, mattress overlay, etc.), so as to avoid the need for separate application of an EMS device and another therapy device.

While a number of embodiments are presented herein, multiple variations of such embodiments, and combinations of elements from one or more embodiments, are possible and are contemplated to be within the scope of the present disclosure. Features enumerated above have been described within the context of particular embodiments. However, as one of ordinary skill in the art would understand, features and aspects of each embodiment may be combined, added to other embodiments, subtracted from an embodiment, etc. in any manner suitable to assist with electrical muscle stimulation. Moreover, those skilled in the art will appreciate that the conception upon which this disclosure is based may readily be used as a basis for designing other devices, assemblies, methods, and systems for carrying out the several purposes of the present disclosure.

## Claims

1. An electrical muscle stimulation system (100), comprising:
at least one electrode (105) configured to transmit an electrical current;
at least one sensing device (110) configured to detect a physiological characteristic of a user; and
at least one controller (120) in communication with the at least one electrode and the at least one sensing device, the at least one controller is configured to:
activate the at least one electrode in response to the at least one sensing device detecting the physiological characteristic of the user to deliver the electrical current at a high frequency toward a target site on the user;
determine a presence of injured tissue at the target site in response to the at least one sensing device detecting a reflected signal;
determine a location of the target site relative to the user based on a measurement of the reflected signal; and
deliver the electrical current with a first charge sufficient to stimulate the target site at the location.

2. The electrical muscle stimulation system of claim 1, wherein the at least one controller is configured to:
activate the at least one electrode to deliver the electrical current to the target site in response to determining the physiological characteristic detected by the at least one sensing device is below a predetermined threshold; and
deactivate the at least one electrode to cease delivery of the electrical current to the target site after a predefined interval.

3. The electrical muscle stimulation system of claim 1, further including a wicking pad (404) coupled to the at least one electrode and the at least one sensing device, the wicking pad includes an interior surface that secures the at least one electrode and the at least one sensing device to the user;
wherein the wicking pad is configured transfer moisture from the interior surface of the wicking pad that is in contact with the user to an exterior surface of the wicking pad, thereby drawing moisture away from the user.

4. The electrical muscle stimulation system of claim 1, further including a garment (420) having a body that is sized and shaped to be worn by the user, wherein the at least one electrode and the at least one sensing device is coupled to the body;
wherein the body is formed of a wicking material such that the garment is configured to remove moisture from the user when the garment is worn by the user.

5. The electrical muscle stimulation system of claim 1, further including a mattress pad (500) having a body that is sized and shaped to receive the user, the mattress pad includes a plurality of electrodes and a plurality of sensing devices on the body;
wherein the at least one controller is configured to selectively activate at least a subset of the plurality of electrodes when a corresponding subset of the plurality of sensing devices detects a pressure along the body when the user is received on the mattress pad.

6. A method for detecting a deep tissue injury, comprising:
transmitting an electrical impulse having a high frequency between a pair of electrodes;
detecting a reflected signal in response to transmitting the electrical impulse;
determining one or more properties of the reflected signal; and
calculating a location of the deep tissue injury relative to at least one of the pair of electrodes.

7. The method of claim 6, wherein the one or more properties of the reflected signal includes an amplitude of the reflected signal or a duration between transmitting the electrical impulse and detecting the reflected signal.

## Patentansprüche

1. Elektrisches Muskelstimulationssystem (100), umfassend:
mindestens eine Elektrode (105), die dazu konfiguriert ist, einen elektrischen Strom zu übertragen;
mindestens eine Erfassungsvorrichtung (110), die dazu konfiguriert ist, eine physiologische Eigenschaft eines Benutzers zu erkennen; und
mindestens ein Steuergerät (120) in Kommunikation mit der mindestens einen Elektrode und der mindestens einen Erfassungsvorrichtung, wobei das mindestens eine Steuergerät zu Folgendem konfiguriert ist:
Aktivieren der mindestens eine Elektrode als Reaktion darauf, dass die mindestens eine Erfassungsvorrichtung die physiologische Eigenschaft des Benutzers erkennt, um den elektrischen Strom mit einer hohen Frequenz an eine Zielstelle am Benutzer zu liefern;
Bestimmen eines Vorhandenseins von verletztem Gewebe an der Zielstelle als Reaktion darauf, dass die mindestens eine Erfassungsvorrichtung ein reflektiertes Signal erkennt;
Bestimmen einer Position der Zielstelle relativ zu dem Benutzer basierend auf einer Messung des reflektierten Signals; und
Liefern des elektrischen Stroms mit einer ersten Ladung, die ausreicht, um die Zielstelle an der Position zu stimulieren.

2. Elektrisches Muskelstimulationssystem nach Anspruch 1, wobei das mindestens eine Steuergerät zu Folgendem konfiguriert ist:
Aktivieren der mindestens einen Elektrode, um den elektrischen Strom an die Zielstelle als Reaktion darauf zu liefern, dass bestimmt wird, dass die von der mindestens einen Erfassungsvorrichtung erkannte physiologische Eigenschaft unter einem vorbestimmten Schwellenwert liegt; und
Deaktivieren der mindestens eine Elektrode, um die Lieferung des elektrischen Stroms an die Zielstelle nach einem vordefinierten Intervall einzustellen.

3. Elektrisches Muskelstimulationssystem nach Anspruch 1, ferner beinhaltend ein Aufsaugkissen (404), das an die mindestens eine Elektrode und die mindestens eine Erfassungsvorrichtung gekoppelt ist, wobei das Aufsaugkissen eine Innenfläche beinhaltet, die die mindestens eine Elektrode und die mindestens eine Erfassungsvorrichtung an dem Benutzer befestigt;
wobei das Aufsaugkissen dazu konfiguriert ist, Feuchtigkeit von der Innenfläche des Aufsaugkissens, die in Kontakt mit dem Benutzer steht, auf eine Außenfläche des Aufsaugkissens zu übertragen, wodurch Feuchtigkeit von dem Benutzer weggezogen wird.

4. Elektrisches Muskelstimulationssystem nach Anspruch 1, ferner beinhaltend ein Kleidungsstück (420) mit einem Körper, das so bemessen und geformt ist, dass es von dem Benutzer getragen werden kann, wobei die mindestens eine Elektrode und die mindestens eine Erfassungsvorrichtung an den Körper gekoppelt sind;
wobei der Körper aus einem aufsaugenden Material so gebildet ist, dass das Kleidungsstück dazu konfiguriert ist, Feuchtigkeit von dem Benutzer zu entfernen, wenn das Kleidungsstück von dem Benutzer getragen wird.

5. Elektrisches Muskelstimulationssystem nach Anspruch 1, ferner beinhaltend eine Matratzenauflage (500) mit einem Körper, die so bemessen und geformt ist, dass sie den Benutzer aufnehmen kann, wobei die Matratzenauflage eine Vielzahl von Elektroden und eine Vielzahl von Erfassungsvorrichtungen an dem Körper beinhaltet;
wobei das mindestens eine Steuergerät dazu konfiguriert ist, mindestens eine Teilmenge der Vielzahl von Elektroden selektiv zu aktivieren, wenn eine entsprechende Teilmenge der Vielzahl von Erfassungsvorrichtungen einen Druck entlang des Körpers erkennt, wenn der Benutzer auf der Matratzenauflage aufgenommen wird.

6. Verfahren zum Erkennen einer tiefen Gewebeverletzung, umfassend:
Übertragen eines elektrischen Impulses mit einer hohen Frequenz zwischen einem Paar Elektroden;
Erkennen eines reflektierten Signals als Reaktion auf das Übertragen des elektrischen Impulses;
Bestimmen einer oder mehrerer Eigenschaften des reflektierten Signals; und
Berechnen einer Position der tiefen Gewebeverletzung relativ zu mindestens einem des Paars Elektroden.

7. Verfahren nach Anspruch 6, wobei die eine oder mehreren Eigenschaften des reflektierten Signals eine Amplitude des reflektierten Signals oder eine Dauer zwischen dem Übertragen des elektrischen Impulses und dem Erkennen des reflektierten Signals beinhalten.

## Revendications

1. Système de stimulation musculaire électrique (100), comprenant :
au moins une électrode (105) conçue pour transmettre un courant électrique :
au moins un dispositif de détection (110) conçu pour détecter une caractéristique physiologique d'un utilisateur ; et
au moins un dispositif de commande (120) en communication avec l'au moins une électrode et l'au moins un dispositif de détection, l'au moins un dispositif de commande est conçu pour :
activer l'au moins une électrode en réponse à la détection par l'au moins un dispositif de détection de la caractéristique physiologique de l'utilisateur pour délivrer le courant électrique à une fréquence élevée vers un site cible sur l'utilisateur ;
déterminer une présence de tissu blessé au niveau du site cible en réponse à la détection par l'au moins un dispositif de détection d'un signal réfléchi ;
déterminer un emplacement du site cible par rapport à l'utilisateur sur la base d'une mesure du signal réfléchi ; et
fournir le courant électrique avec une première charge suffisante pour stimuler le site cible au niveau de l'emplacement.

2. Système de stimulation musculaire électrique de la revendication 1, ledit au moins un dispositif de commande étant conçu pour :
activer l'au moins une électrode pour délivrer le courant électrique au site cible en réponse à la détermination que la caractéristique physiologique détectée par l'au moins un dispositif de détection est inférieure à un seuil préétabli ; et
désactiver l'au moins une électrode pour cesser de délivrer le courant électrique au site cible après un intervalle prédéfini.

3. Système de stimulation musculaire électrique de la revendication 1, comprenant en outre un tampon à effet de mèche (404) couplé à l'au moins une électrode et à l'au moins un dispositif de détection, le tampon à effet de mèche comprend une surface intérieure qui fixe l'au moins une électrode et l'au moins un dispositif de détection à l'utilisateur ;
ledit tampon à effet de mèche étant conçu pour transférer l'humidité de la surface intérieure du tampon à effet de mèche qui est en contact avec l'utilisateur à une surface extérieure du tampon à effet de mèche, éloignant ainsi l'humidité de l'utilisateur.

4. Système de stimulation musculaire électrique de la revendication 1, comprenant en outre un vêtement (420) possédant un corps qui est dimensionné et façonné pour être porté par l'utilisateur, ladite au moins une électrode et ledit au moins un dispositif de détection étant couplés au corps ;
ledit corps étant formé d'un matériau à effet de mèche de sorte que le vêtement soit conçu pour retirer l'humidité de l'utilisateur lorsque le vêtement est porté par l'utilisateur.

5. Système de stimulation musculaire électrique de la revendication 1, comprenant en outre un couvre-matelas (500) possédant un corps qui est dimensionné et façonné pour recevoir l'utilisateur, le couvre-matelas comprend une pluralité d'électrodes et une pluralité de dispositifs de détection sur le corps ;
ledit au moins un dispositif de commande étant conçu pour activer sélectivement au moins un sous-ensemble de la pluralité d'électrodes lorsqu'un sous-ensemble correspondant de la pluralité de dispositifs de détection détecte une pression le long du corps lorsque l'utilisateur est reçu sur le couvre-matelas.

6. Procédé permettant la détection d'une blessure tissulaire profonde, comprenant :
la transmission d'une impulsion électrique possédant une fréquence élevée entre une paire d'électrodes ;
la détection d'un signal réfléchi en réponse à la transmission de l'impulsion électrique ;
la détermination d'une ou de plusieurs propriétés du signal réfléchi ; et
le calcul d'un emplacement de la blessure tissulaire profonde par rapport à au moins l'une de la paire d'électrodes.

7. Procédé de la revendication 6, ladite ou lesdites propriétés du signal réfléchi comprenant une amplitude du signal réfléchi ou une durée entre la transmission de l'impulsion électrique et la détection du signal réfléchi.
